# EUROPEAN PATENT SPECIFICATION

(11) **EP 0 454 728 B1**
(45) Date of publication and mention of the grant of the patent: **16.03.1994**
(21) Application number: 90901973.9
(22) Date of filing: 11.12.1989
(51) Int. Cl.: C07D 311/30, A61K 31/35

(54) **FLUORINATED FLAVONE ACETIC ACID**
FLUORIERTE FLAVON-ESSIGSÄURE
ACIDE ACETIQUE DE FLAVONE FLUORURE

(30) Priority: 19.01.1989 US 299106
(43) Date of publication of application: 06.11.1991
(73) Proprietor: THE UPJOHN COMPANY, Kalamazoo, Michigan 49001 (US)
(72) Inventor: ARISTOFF, Paul, A., Portage, MI 49002 (US)
(74) Representative: Perry, Robert Edward
(86) International application number: US8905446
(87) International publication number: WO9008143

(56) References cited:
- EP-A- 0 080 934
- Patent Abstracts of Japan, vol. 9, no. 153 C-288)(1876), 27 June 1985

## Description

### Background of the Invention

The present invention is directed toward novel fluorinated flavone acetic acids (FAA) suitable for use as antitumor agents. The fluorinated FAA compounds are more effective than their unfluorinated counterparts. For example B. Derwinko and L-Y. Yang, "The Activity of flavone acetic acid (NSC 347512) on human colon cancer cells in vitro" Invest. New Drugs, 4:289-93 (1986) disclose that FAA (4-oxo-2-phenyl-4H-1-benzopyran-8-acetic acid) had "relatively poor cytotoxic effects and because the therapeutic range of FAA is so narrow, we conclude that this agent will not be a valuable contribution to the antitumor arsenal at least for colon cancer." Meanwhile, the fluorinated FAA of the present invention has shown curative activity in murine pancreatic carcinoma models and against solid tumors. In particular, they have been shown to exhibit activity against human tumor cell lines and an FAA resistant cell line.

### Information Disclosure

U.S. Patents 4,783,533 and 4,602,034 disclose FAA compounds chemically called (4-oxo-4H-(1)-benzopyran-8-yl)alconic acids their salts and derivatives as well as methods for their preparation and use in the control of tumors. European Patent Application 0278176 discloses various flavone acetic acid analogs described as xanthenone-4-acetic acid. The synthesis and antitumor effect of substituted 4-oxo-4H-1-benzopyrans are described in Eur. J. Med. Chem., 20, 5, p. 393-402 (1985) which was authored by the same inventors of 4,783,533 and 4,602,034.

FAA compounds, particularly a diethylaminoethyl ester derivative are discussed in Drugs of the Future, Vol. 12, 2, p. 123-25 (1987). An article in Cancer Research, 48, 5878-82 (October 15, 1988) entitled "Phase I and Clinical Pharmacology Study of Intravenous Flavone Acetic Acid" by Weiss, et al. describes the basic antitumor effects of FAA and results when administered to patients. FAA is reported to have murine tumor activity in animals although no objective tumor response was observed in humans tested. The article also reports the toxicity problems associated with FAA such as acute hypotension and generalized fatigue and asthemia.

### Summary of the Invention

In one aspect the subject invention is a compound according to Formula I wherein R₁ is CHO, CN, CO₂M, CO₂R₃ or CONR₃R₄ where M is hydrogen or a pharmaceutically acceptable salt such as Li, Na, K, Ca or other acceptable counter-ion for carboxylic acids, R₃ and R₄ are independently hydrogen, C₁-C₁₂ alkyl or heterosubstituted alkyl, C₃-C₁₀ cycloalkyl or heterosubstituted cycloalkyl, C₆-C₁₂ aryl, C₇-C₂₄ alkylaryl or are joined to form a C₃-C₁₀ cycloalkyl or heterosubstituted cycloalkyl; R₂ is hydrogen, fluorine, methyl, CF₃, phenyl or substituted phenyl. The fluorinated phenyl ring can be fluorinated at any of the C2 to C6 positions and n is 1 to 5, inclusive. Preferably, R₁ is CO₂M and R₂ is hydrogen.

Typical fluorinated phenyl rings can include 3-fluorophenyl, 2,3,4,5-tetrafluorophenyl, pentafluorophenyl, 3,4-difluorophenyl, 2-fluorophenyl, 4-fluorophenyl, 2,6-difluorophenyl, 3,5-difluorophenyl, 3,4,5-trifluorophenyl, 2,3,5,6-tetrafluorophenyl, 2,3-difluorophenyl, 2,3,4-trifluorophenyl, 2,4,6-trifluorophenyl, 2,5-difluorophenyl or 2,4-difluorophenyl. Preferred compounds include 4-oxo-2-(3-fluorophenyl)-4H-1-benzopyran-8-acetic acid, 4-oxo-2-(pentafluorophenyl)-4H-1-benzopyran-8-acetic acid, 4-oxo-2-(3,4-difluorophenyl)-4H-1-benzopyran-8-acetic acid, 4-oxo-2-(2-fluorophenyl)-4H-1-benzopyran-8-acetic acid, 4-oxo-2-(4-fluorophenyl)-4H-1-benzopyran-8-acetic acid, 4-oxo-2-(2,6-difluorophenyl)-4H-1-benzopyran-8-acetic acid, or 4-oxo-2-(3,5-difluorophenyl)-4H-1-benzopyran-8-acetic acid, 4-oxo-2-(3,4-difluorophenyl)-4H-1-benzopyran-8-acetic acid, 4-oxo-2-(pentafluorophenyl)-4H-1-benzopyran-8-acetic acid, 4-oxo-2-(3,4,5-trifluorophenyl)-4H-1-benzopyran-8-acetic acid, 4-oxo-2-(2,3,5,6-tetrafluorophenyl)-4H-1-benzopyran-8-acetic acid, 4-oxo-2-(2,3-difluorophenyl)-4H-1-benzopyran-8-acetic acid, 4-oxo-2-(2,3,4-trifluorophenyl)-4H-1-benzopyran-8-acetic acid, 4-oxo-2-(2,4,6-trifluorophenyl)-4H-1-benzoypran-8-acetic acid, 4-oxo-2-(2,5-difluorophenyl)-4H-1-benzopyran-8-acetic acid or 4-oxo-2-(2,4-difluorophenyl)-4H-1-benzopyran-8-acetic acid.

In another aspect the subject invention is directed toward a pharmaceutical composition comprising an effective amount of a compound according to Formula I descibed above and a pharmaceutically acceptable carrier.

In yet another aspect the subject invention is directed toward an antitumor composition comprising an antitumor effective amount of a compound according to Formula I and a pharmaceutically acceptable carrier.

Generally, the fluorinated flavone acetic acid compounds have been discovered to have antitumor activity superior to unflourinated flavone acetic acid compounds. In addition, the fluorinated flavone acetic acids appear to have less toxic effects in animals than the unfluorinated flavone acetic acids.

### Detailed Description of the Invention

The present invention is directed to fluorinated FAA compounds represented by the structural formula I. Wherein R₁ is CHO, CN, CO₂M, CO₂R₃ or CONR₃R₄ where M is hydrogen or a pharmaceutically acceptable salt such as Li, Na, K, Ca or other acceptable counter-ion for carboxylic acids, R₃ and R₄ are independently hydrogen, C₁-C₁₂ alkyl or heterosubstituted alkyl, C₃-C₁₀ cycloalkyl or heterosubstituted cycloalkyl, C₆-C₁₂ aryl, alkylaryl or where they are joined to form a C₃-C₁₀ cycloalkyl or heterosubstituted cycloalkyl (and are attached to the proximate nitrogen). R₂ is hydrogen, fluorine, methyl, CF₃, phenyl or substituted phenyl. NR₃R₄ can be morpholino, piperidino, diethylaminoethyl or dimethylaminoethyl.

The fluorinated phenyl ring can be fluorinated at any of the C2 to C6 positions and n is 1 to 5 inclusive. Typical fluorinated phenyl ring structures can include 3-fluorophenyl, 2,3,4,5-tetrafluorophenyl, pentafluorophenyl, 3,4-difluorophenyl, 2-fluorophenyl, 4-fluorophenyl, 2,6-difluorophenyl, 3,5-difluorophenyl, 3,4,5-trifluorophenyl, 2,3,5,6-tetrafluorophenyl, 2,3-difluorophenyl, 2,3,4-trifluorophenyl, 2,4,6-trifluorophenyl, 2,5-difluorophenyl or 2,4-difluorophenyl.

Examples of "C₁-C₁₂ alkyl" are one to twelve carbon atom chains from methyl to dodecyl and isomeric forms thereof. Examples of "C₃-C₁₀ cycloalkyl" are three to ten carbon atoms formed in a ring such as cyclopropyl, cyclobutyl, cyclopentyl and cyclohexyl. Examples of "aryl" are six to twelve carbon atom rings which can be substituted with one to three hydroxy, C₁-C₃ alkyl, trifluoromethyl, fluoro, chloro or bromo group such as phenyl, α-naphthyl, β-naphthyl, m-methylphenyl, p-trifluoromethylphenyl and the like. "Alkylaryl" are seven to twenty-four carbon atoms from a C₁-C₁₂ alkyl and a C₆-C₁₂ aryl as defined.

The "heterosubstituted" forms of the alkyl and cycloalkyl groups are when a carbon in the chain or ring structure is replaced by a heteroatom such as nitrogen, oxygen or sulfur, for example, piperidino and morpholino. The heteroatom can then contain further alkyl, aryl or cycloalkyl groups to complete the valence, for example, dimethylaminoethyl or diethylaminoethyl. "Substituted phenyl" is a phenyl ring having a substituent pending therefrom such as fluorine, chlorine or bromine, hydroxy, or a C₁-C₄ alkyl group.

These compounds can be prepared by one or more methods described below as well as in accordance with the synthesis schemes disclosed in U.S. Patents 4,783,533 and 4,602,034, utilizing the appropriate fluorinated phenyl ring as depicted in Formula I.

Generally the fluorinated FAA's of the present invention can be prepared as depicted in Schemes 1 or 2, below. In both Schemes 1 and 2, the various steps are well known in the art. That is, in Scheme 1, step 1 involves a Claisen-condensation of the β-ketoesters. Step 2 is a Simonis-type reaction, acid catalyzed β-ketoester condensation, such as with polyphosphoric acid, or phosphoric acid and phosphorous pentoxide. Step 3 is a radical catalyzed benzylic bromination, such as with N-bromosuccinimide in carbon tetrachloride. Step 4 involves the conversion of bromide to nitrile, a displacement using potassium cyanide and potassium iodide. Finally, Step 5 is the hydrolysis of nitrile to acid using, for example, acetic acid, water and sulfuric acid.

In Scheme 2, step 1 is a phenol alkylation which can employ allyl bromide and a base such as potassium carbonate or potassium hydroxide. Step 2 is Claisen rearrangement either by heating or by a Lewis acid treatment. Step 3 is an Allan-Robinson type reaction to prepare flavones such as disclosed in P.K. Jain, et al., Synthesis, pp. 221-22 (1982). Step 4 is an oxidation of the allyl group directly to an acid, for example by using RuCl₃, NaIO₄ or NaIO₄, KMnO₄ or ozonolysis followed by ozonide oxidation with hydrogen peroxide. Step 5 is the oxidation of the allyl group to an aldehyde. This can be accomplished by ozonolysis or osmium tetroxide/sodium periodate or with RuCl₃/NaIO₄. Finally, Step 6 is the oxidation of the aldehyde to an acid such as by sodium chlorite oxidation or silver oxide oxidation.

The fluorinated FAA compounds are more effective as antitumor agents than their unfluorinated analogs as is demonstrated by the in vitro and in vivo experiments. The subject compounds can be effectively administered intraperitoneally, orally, subcutaneously or intravenously. A pharmaceutical composition of this invention contains as its active ingredient the fluorinated FAA compound associated or admixed with an acceptable vehicle or pharmaceutical excipient in suitable form for administration.

Unit doses may be sugar-coated pills, tablets, capsules, gellules, phials or bottles. The dosage forms contain between 50 and 1000 mg of active ingredient.

As an example, the following compositions can be formulated: Coated pill: active ingredient: 100 mg. Excipients:magnesium stearate, lactose, talcum, starch, alginic acid, hydroxypropylcellulose. Bottle: active ingredient: 1000 mg in freeze-dried form desolved in 20 ml of water for administration by injection.

The preferred doses are 1 mg/kg to 300 mg/kg by bolus injection and 0.02 mg/kg/min to 60 mg/kg/min by infusion. Of course, the dose will vary depending upon the age, weight, route of administration and physical condition of the recipient.

Pharmacological tests have been carried out on several types of tumor cells in a disk diffusion assay see, generally T.H. Corbett, et al., In Vitro and In Vivo Models of Detection of New Antitumor Drugs, L.J. Hanku, T. Konda and B.J. White, ed., Univ of Tokyo Press, pages 5-14 (1986). The fluorinated FAA (4-oxo-2-(3-fluorophenyl)-4H-1-benzopyran-8-acetic acid) of Example 1 was tested against human H125 lung cells and CX-1 colon cells, mouse cell lines C09, P03 and C38, leukemia L1210 cells and FAA resistant cell lines at various doses. The results are shown in the Table 1, below. A larger number indicates a greater zone of inhibition and thus more antitumor effectiveness.

**TABLE 1**

| Test | Dose µg/disk | Leukemia L1210 | Mouse | | | Human | | FAA Resistant |
|---|---|---|---|---|---|---|---|---|
| | | | CO9 | PO3 | C38 | H125 | CX-1 | |
| 1 | 2000 | 460 | 600 | - | - | - | 320 | - |
| 2 | 1000 | 260 | - | 280 | 150 | 200 | 0 | 240 |
| 3 | 500 | 140 | - | 220 | 240 | 0 | - | 200 |
| 4 FAA | 1000 | 400-520 | - | 600-950 | - | 0 | 0 | 0 |
| 5 FAA | 500 | 0-40 | - | 240 | 400 | 0 | 0 | 0 |

Table 1 shows the fluorinated FAA compound (Tests 1-3) to have a dose related anti-tumor activity against leukemia L1210 cells and the various mouse and human cell lines. Especially interesting is the activity demonstrated against the normally FAA resistant cell line at the 1000 and 500 µg/disk dosages and, in particular, against human colon cell line CX-1 at 2000 µg/disk and the human lung line H125 at 1000 µg/disk dosages. Also, it is demonstrated that the FAA (4-oxo-2-phenyl-4H-1-benzopyran-8-acetic acid) controls (Tests 4-5) showed no activity against the human cell lines and the FAA resistant cell line. It is also recognized that while the FAA controls show activity against the mouse cell lines they have been known to not show activity against human cell lines as demonstrated here.

In a separate experiment the fluorinated FAA compound of Example 1 was tested for cytotoxicity against murine and human tumor cells in the disk diffusion assay. These results are shown in Table 2. Each disk was treated with 1000 µg per disk of the fluorinated FAA compound.

**TABLE 2**

| Test | Mouse | | | FAA Resistant Cell Line | Human | | | |
|---|---|---|---|---|---|---|---|---|
| | L1210 | Colon 08 | Colon 07 | | Colon 116 | Colon CX-1 | HCT8 | Lung H125 |
| 1 | 440 | 600 | 600 | 350 | 370 | 370 | - | 370 |
| 2 | 270-320 | 600 | 240 | 230 | 320 | - | 280 | - |

The results in Table 2 indicate that the subject compound of Example 1 had good activity in vitro against three human tumor colon cell lines (116, CX-1 and HCT8) and a human tumor lung line, H125.

The fluorinated FAA compound of Example 1 was also tested in vivo in mice. Escalating dosages were administered to mice having colon adenocarcinoma 38. The maximum dosage tolerated per IV injection was between 150 to 220 mg/kg. The first dosage of about 100 mg/kg produced a stupor but subsequent dosages escalated to 150 mg/kg were well tolerated. The fluorinated FAA (tests 1 and 2) was obviously active against Colon 38 as shown in Table 3, below, versus a Control which was tumored mice receiving no drug treatment.

**TABLE 3**

| Test | # Mice | Dosage mg/kg | Days Injected | Drug Deaths | Median Tumor mg/on day 31 | T/C in % | Tumor Free at day 60 |
|---|---|---|---|---|---|---|---|
| 1 | 5 | 220 | 3 | 3 | 44 | 3 | 1 |
| 2 | 5 | 150 | 6,9,12,15 | 0 | 448 | 32 | 2 |
| Control | 6 | 0 | - | 0 | 1383 | - | 0 |

Additionally, BDF₁ mice were treated for early stage pancreatic ductal adenocarcinoma 03. These tests are shown in Tables 4 and 5 below.

**TABLE 4**

| Test | Drug | Dose mg/kg/injection | Total Dosage | Drug Deaths | Tumor Free on Day 26 |
|---|---|---|---|---|---|
| 1 | FAA | 235 | 205 | 5/5 | Toxic |
| 2 | FAA | 162 | 486 | 1/5 | 4/5 |
| 3 | FAA | 112 | 336 | 0/5 | 1/5 |
| 4 | Ex. 1* | 155 | 465 | 4/6 | 2/6 |
| 5 | Ex. 1* | 107 | 321 | 0/6 | 3/6 |
| 6 | Ex. 1* | 74 | 222 | 0/5 | 0/5 |
| 7 | Control | 0 | 0 | 0/6 | 0/6 |

| | | | | | |
|---|---|---|---|---|---|
| *Compound of Example 1, 4-oxo-2-(3-fluorophenyl)-4H-1-benzopyran-8-acetic acid. | | | | | |

The data in Table 4 indicates that at the lower dosage level of 321 mg/kg the fluorinated FAA was more potent as an antitumor drug than FAA.

**TABLE 5**

| Test | Drug | Dose mg/kg/injection | Total Dosage | Drug Deaths | Tumor Free on Day 26 |
|---|---|---|---|---|---|
| 1 | Ex.4* | 80 on day 3 | 330 | 0/5 | 3/5 |
| | | 100 on day 5 | | | |
| | | 150 on day 7 | | | |
| 2 | Ex. 5** | 90 on day 3 | 360 | 0/5 | 4/5 |
| | | 110 on day 5 | | | |
| | | 160 on day 7 | | | |

| | | | | | |
|---|---|---|---|---|---|
| * Compound of Example 4, 4-oxo-2-(2-fluorophenyl)-4H-1-benzopyran-8-acetic acid. | | | | | |
| ** Compound of Example 5, 4-oxo-2-(4-fluorophenyl)-4H-1-benzopyran-8-acetic acid. | | | | | |

Both drugs used in tests 1 and 2 were effective in the treatment of tumors. Interesting was that these drugs did not cause a stupor even at the higher dosage injections. The mice were repeatedly checked up to 3 hours post injection and no stupor was observed. This is in sharp contrast to FAA and even the Example 1 compound which showed modest stupor in mice after higher dose injections of about 180 mg/kg.

For example, the fluorinated FAA of Example 1 produced shallow rapid breathing and modest stupor. The dosages were escalated to produce evident toxicity. At about 180 mg/kg one out of five mice tested were killed. The compound showed activity against tumors as three out of five mice were tumor free on the 35th day. The remaining mouse had a very small mass of 32 mg which may not have been viable tumor cells. Meanwhile, in the control group where no treatment was given all six mice exhibited a median tumor of 1852 grams on the 28th day and were not tumor free on the 35th day.

### Example 1: Preparation of 4-oxo-2-(3-fluorophenyl)-4H-1-benzopyran-8-acetic acid.

### Step 1

A mixture of 60 g of o-cresol and 153 g of ethyl 3-fluorobenzoylacetate was added over 30 minutes to a mechanically stirred solution of 750 g of polyphosphoric acid. The resulting mixture was stirred at 75° C for 4 hours, then cooled and poured into 8 liters of ice water and subsequently extracted with three four liter portions of ethyl acetate. The solvent in the organic fractions was concentrated in vacuo and the residue chromatographed over 7 kg of silica gel eluting with 20% acetone in hexane to give 23 g of title product which was then recrystallized from ethyl acetate to give 16 g of pure 8-methyl-2-(3-fluorophenyl)-4H-benzopyran-4-one. TLC Rf 0.3 in 10% acetone in hexane; NMR (CDCl₃) δ 2.6 (s, 3H), 6.8 (s, 1H), 8.04-8.08 (m, 1H).

### Steps 2 & 3

A mixture of 14.3 g of 8-methyl-2-(3-fluorophenyl)-4H-benzopyran-4-one, 17.2 g of N-bromosuccinimide and 3.5 g of azobisisobutyronitrile (AIBN) in 700 ml of carbon tetrachloride was refluxed for 6.5 hours, cooled to room temperature and stirred overnight and then diluted with 100 ml of water. The solvents were removed in vacuo and the residue recrystallized from hot ethyl acetate to give 16.5 g of 8-bromomethyl-2-(3-fluorophenyl)-4H-benzpyran-4-one which was used without further purification.

A suspension of 11.2 g of the above prepared 8-bromomethyl-2-(3-fluorophenyl)-4H-benzopyran-4-one, 3.2 g of potassium cyanide, 5.7 g of potassium iodide and 1.3 g of aliquot 336 (tricaprylylmethylammonium chloride) in 45 ml of water and 485 ml of toluene was stirred for 25.5 hours at 73° C, cooled, diluted with water and extracted with methylene chloride. The solvents in the organic layer were concentrated in vacuo and the resulting residue chromatographed over silica gel with 3-5% acetone in a 1:1 mixture of methylene chloride and hexane to give 4.6 g of 8-cyanomethyl-2-(3-fluorophenyl)-4H-benzopyran-4-one. IR 1643 cm⁻¹; NMR (CDCl³): δ 4.06 (2, 1H), 6.8 (s, 1H), 7.0-7.85 (m, 6H), 8.2 (m, 1H).

### Step 4

A mixture of 4.6 g of 8-cyanomethyl-2-(3-fluorophenyl)-4H-benzopyran-4-one; 18 ml of glacial acetic acid and 18 ml of sulfuric acid in 418 ml of water was stirred 12 hours at room temperature and diluted with 200 ml of water. The resulting suspension was filtered and the solids washed with water, then dissolved in 170 ml of 5% aqueous sodium bicarbonate, filtered to remove insoluble solids and acidified by addition of 11 ml of concentrated sulfuric acid. The resulting precipitate was filtered and the solids washed with water and dried to give 4.4 g of 4-oxo-2-(3-fluorophenyl)-4H-1-benzopyran-8-acetic acid as a white solid: IR 1645 and 1700 cm⁻¹; m.p. 208-211° C.

### Example 2: Preparation of 4-oxo-2-(3,4-difluorophenyl)-4H-1-benzopyran-8-acetic acid.

### Step 1

A suspension of 6.2 g of potassium hydroxide, 10 g of 2-hydroxyacetophenone and 17.8 g of allyl bromide in 550 ml of acetone was refluxed for 16 hours, cooled and filtered. The solids were extracted with three 250 ml portions of chloroform and the combined acetone filtrate and chloroform extracts concentrated to give a residue which was then heated at 165° C for 18 hours, cooled, diluted with 250 ml of hexane and filtered. The resulting filtrate was concentrated in vacuo to give 10.02 g of 3'-allyl-2'-hydroxyacetophenone as a liquid: TLC Rf 0.70 in 3:1 toluene-methyl isobutylketone; C-13 NMR (CDCl₃): δ 26.72, 33.41, 116.0, 118.44, 119.21, 128.82, 129.34, 136.10, 136.44, 160.40, 204.75; ¹H-NMR (CDCl₃): δ 2.60 (s, 3H), 3.40 (d, J=6.4 Hz, 2H); 5.05 (d, J=1.4 Hz, 1H), 5.08-5.11 (m, 1H), 5.92-6.06 (m, 1H), 6.82 (t, J=7.6 Hz, 1H), 7.33 (d, J-6.7 Hz, 1H), 7.59 (d, J=6.7 Hz, 1H), 12.62 (s, 1H).

### Step 2

A mixture of 1.7 g (9.4 mmol) of 3'-allyl-2'-hydroxyacetophenone, 2.0 g (11 mmol) of 3,4-difluorobenzoyl chloride, 1.6 g (4.7 mmol) of tetra-n-butylammonium bisulfate, 60 ml of 10% aqueous potassium hydroxide and 60 ml of benzene was heated at 60° C for 4 hours, cooled and the phases separated. The organic phase was washed with three 60 ml portions of water, treated with 5.6 g (28 mmol) of p-toluenesulfonic acid monohydrate and an additional 60 ml of benzene and refluxed for 3 hours, using a Dean-Stark trap to remove the water in the reaction. The resulting mixture was washed with 150 ml of 8% aqueous sodium bicarbonate and then with three 60 ml portions of water. The organic phase was then concentrated in vacuo; treated with 25 ml of methanol and cooled to -20° C. After four hours the resulting solid was filtered, washed with three 5 ml portions of methanol and dried to give 1.62 g (56%) of 8-allyl-2-(3,4-difluorophenyl)-4H-benzopyran-4-one as a solid: m.p. 127-130° C; TLC Rf 0.48 in 3:1 toluene-methyl isobutyl ketone.

### Step 3

A mixture of 1.0 g (3.4 mmol) of 8-allyl-2-(3,4-difluorophenyl)-4H-benzopyran-4-one, 3.6 g (17 mmol) of sodium periodate, 7 ml of carbontetrachloride, 7 ml of acetonitrile and 15 ml of water was treated with 0.14 g (0.66 mmol) of rothenium (III) chloride, stirred for 4 hours and then diluted with 50 ml of methylene chloride. The phases were separated and the aqueous phase extracted with three 50 ml portions of methylene chloride. The combined organic phases were concentrated in vacuo, treated with 500 ml of diethyl ether and filtered. The resulting solids were extracted with three 100 ml portions of 8% aqueous sodium bicarbonate solution and the aqueous extract washed with three 100 ml portion of chloroform. The aqueous phase was treated with concentrated hydrochloric acid (to pH 1) and then extracted with three 100 ml portions of chloroform. The final chloroform extract was evaporated in vacuo to give 0.131 g (12%) of 4-oxo-2-(3,4-difluorophenyl)4H-1-benzopyran-8-acetic acid as a solid: m.p. 145-147° C; TLC Rf 0.26 in 64:25:10:1 toluene-methylisobutyl ketone-methanol-acetic acid.

### Example 3: Preparation of 4-oxo-2-(pentafluorophenyl)-4H-1-benzopyran-8-acetic acid.

### Step 1

A mixture of 19 g (mmol) 3'-allyl-2'-hydroxyacetophenone, 25 g (110 mmol) pentafluorobenzoyl chloride, 36 g (110 mmol) tetra-n-butylammonium bisulfate, 750 ml 10% aqueous KOH, and 750 ml benzene was heated at 80° C for 6 hours, cooled and the phases separated. The organic phase was concentrated in vacuo and flash chromatographed over 100 g silica gel with success, 2 liter portions of hexane, diethyl ether and CHCl₃. The diethyl ether eluate was evaporated in vacuo. The residue was dissolved in 250 ml methanol and cooled at-20° C for 24 hours. The mixture was filtered and yielded 8-allyl-2-(pentafluorophenyl)-4H-benzopyran-4-one as 10.141 g (27%) solid: TLC R_{f} 0.35 in 49:25:25:1 CHCl₃:toluene:hexane:methanol.

### Step 2

A mixture of 2.0 g (5.7 mmol) 8-allyl-2-pentafluorophenyl)-4H-benzopyran-4-one, 5.8 g (27.mmol) NaIO₄, 0.091 g (0.36 mmol) RuCl₃·H₂O, 50 ml CH₃CN, 50 ml CCl₄ and 100 ml H₂O was stirred at room temperature. After 5 hours, 300 ml CHCl₃ was added to the mixture and the phases were separated. The aqueous phase was extracted with 500 ml 9:1 CHCl₃:CH₃OH and 400 ml H₂O was added to the aqueous phase. This aqueous phase was extracted with two 500 ml portions 9:1 CHCl₃:CH₃OH. The combined organic extracts were concentrated in vacuo to approximately 25 m and added to 1 lieter of hexane The mixture was filtered in vacuo and the filtered solids were redissolved and precipitated in hexane two more times. This yielded 0.259 g (12%) of 4-oxo-2-(pentafluorophenyl)-4H-1-benzopyran-8-acetic acid as a solid: m.p. 156-160° C; TLC R_{f} 0.23 in 24:25:10:1 toluene: methylisobutylketone:methanol:acetic acid.

### Example 4: Preparation of 4-oxo-2-(2-fluorophenyl)-4H-1-benzopyran-8-acetic acid.

### Step 1

A mixture of 0.528 g (3 mmol) of 3'-allyl-2'-hydroxyacetophenone, 0.57 g (3.6 mmol) of 2-fluorobenzoyl chloride, 0.509 g (1.5 mmol) of tetra-n-butylammonium bisulfate, 20 ml of 10% aqueous potassium hydroxide and 20 ml of benzene was heated at 80° C for 3 hours. The layers were separated and the benzene layer washed thoroughly with water (3 x 20 ml) and the water removed from the benzene layer by azeotropic distillation. The resulting residue was treated with 1.7 g (9 mmol) of p-toluenesulphonic acid and benzene (25 ml) and heated for two hours with azeotropic removal of water. The benzene solution was washed with 8% aqueous sodium bicarbonate (50 ml) and the solvent evaporated in vacuo. The resulting residue was recrystallized from ethyl acetate to give 0.49 g (58%) of 8-allyl-2-(2-fluorophenyl)-4H-benzopyran-4-one as white solid: M.P. 78-81° C; TLC R_{f} 0.40 in 10:1 hexane-ethylacetate.

### Step 2

A mixture of 1.4 g (5 mmol) of 8-allyl-2-(2-fluorophenyl)-4H-benzopyran-4-one, 4.4 g (20.5 mmol) of sodium periodate, 50 ml of carbon tetrachloride, 50 ml of acetonitrile, and 75 ml of water was treated with 0.025 g (2.2% mol) of ruthenium trichloride hydrate. The entire mixture was stirred vigorously for 2 hours at room temperature and the phases were separated. The upper aqueous phase was extracted with three 50 ml portions of methylene chloride. The combined organic extracts were washed with 30 ml of 10% aqueous sodium bisulfite solution and then extracted with three 50 ml portions of 10% aqueous sodium hydroxide solution. The aqueous phase was treated with concentrated hydrochloride acid (to pH 1) and then extracted with three 100 ml portions of ethyl acetate. The extracts were dried over magnesium sulfate and concentrated in vacuo to give yellow solid which was recrystallized in methanol to give 0.274 g (18%) of 4-oxo-2-(2-fluorophenyl)-4H-1-benzopyran-8-acetic acid as a white solid: M.P. 179-181° C; TLC R_{f} 0.29 in 40:40:1 hexane-ethyl-acetate-acetic acid.

### Example 5: Preparation of 4-oxo-2-(4-fluorophenyl)-4H-1-benzopyran-8-acetic acid.

### Step 1

The same procedure as used in Example 4 was used to prepare this compound. Starting from a 3 mmol scale, 0.375 g (45%) of 8-allyl-2-(4-fluorophenyl)-4H-benzopyran-4-one was obtained as a white solid: M.P. 98-100° C; TLC R_{f} 0.38 in 10:1 hexane-ethyl acetate.

### Step 2

A similar procedure as used in Example 4 was used employing 6 equivalents of sodium periodate and 3.3 mol % of ruthenium trichloride for 1.5 hours at room temperature to give 0.075 g (25%) of 4-oxo-2-(4-fluorophenyl)-4H-1-benzopyran-8-acetic acid as white crystals: M.P. 207-210° C.

### Example 6: Preparation of 4-oxo-2-(2',3',4',5'-tetrafluorophenyl)-4H-1-benzopyran-8-acetic acid.

### Step 1

A mixture of 25 g (130.mmol) 2,3,4,5-tetrafluorobenzoic acid, 0.5 ml DMF, 200 ml CH₂Cl₂ and 17 ml (192.mmol) oxalyl chloride was stirred at room temperature for 21 hours. The mixture was filtered and evaporated in vacuo. The concentrate was extracted with 3 x 300 ml hexane. The extract was filtered via gravity through a coarse pore sintered glass funnel. This filtrate was evaporated in vacuo to yield 22 g (76%) of 2,3,4,5-tetrafluorobenzoylchloride as a colorless liquid. ¹H-NMR(CDCl₃) δ 281-289 (m, 1H); ¹³C-NMR(CDCl₃): δ 114.882 (d, J = 21.7 Hz), 141.300 (d, J = 270.0 Hz), 144.763 (d, J = 279 Hz), 146.303 (d, J = 241 Hz), 147.595 (d, J = 279 Hz), 160.593.

### Step 2

A mixture of 19.3 g (110 mmol) 3'-allyl-2'-hydroxyacetophenoxy 22 g (110 mmol) 2,3,4,5-tetrafluorobenzoyl chloride, 38.4 g (110 mmol) tetra-n-butylammonium bisulfate, 750 ml 10% aqueous KOH and 750 ml benzene was heated at 80° C for 6 hours, cooled and the phases were separated. The organic phase was concentrated in vacuo and flash chromatographed over 100 g silica gel with successive 2 l portions of hexane and 9:1 CHCl₃:CH₃OH. The CHCl₃:CH₃OH eluate was concentrated in vacuo, treated with 500 ml hot CH₃OH and cooled to-20° C. After 24 hours, the resulting solid was filtered, washed with three 25 ml portions of cold methanol and dried to give 5.35 g of 8-allyl-2-(2,3,4,5-tetrafluorophenyl)-4H-benzopyran-4-one as a solid: TLC R_{f} 0.37 in 49:25:25:1 CHCl₃:toluene:hexane:CH₃OH; ¹H-NMR (DMSO) δ 3.44 (d, J = 6.5 Hz, 2H), 5.06 (s, 1H), 5.09-5.10 (m, 1H), 5.92-6.09 (m, 1H), 6.94 (s, 1H), 7.00 (t, J = 9.6 Hz, 1H), 7.31 (d, J = 7.4 Hz, 1H), 7.55 (d, J = 7.8 Hz, 1H), 7.82-7.88 (m, 1H); ¹³(-NMR (DMSO) δ 35.376, 107.823 (d, J = 18.9 Hz), 112.735, 117.685, 120.791, 121.10, 121.686, 129.056, 130.408, 135.025, 138.032, 141.300 (d, J = 270 Hz), 144.763 (d, J = 279 Hz), 146.303 (d = 241 Hz), 147.595 (d, J = 279 Hz), 154.957, 164.706, 176.488.

### Step 3

A mixture of 2.0 g (6.0 mmol) of 8-allyl-2-(2,3,4,5-tetrafluorophenyl)-4H-benzopyran-4-one, 6.4 g (30 mmol) NaIO₄, 0.100 (0.40 mmol) RuCl₃·H₂O, 50 ml CCl₄, 50 ml CH₃CH and 100 ml H₂O was stirred at room temperature. After 5 hours, 300 ml CHCl₃ was added and the phases separated. The aqueous phase was extracted with 500 ml 9:1 CHCl₃:CH₃OH. An additional 400 ml H₂O was added to the aqueous phase which was then extracted with two 500 ml portions of 9:1 CNCl₃:CH₃OH. The combined organic extracts were concentrated in vacuo to approximately 25 ml and added to 1 l of hexane. The mixture was filtered in vacuo and the filtered solids were redissolved and precipitated in hexane two more times. This yielded 0.345 g of 4-oxo-2-(2,3,4,5-tetrafluorophenyl-4H-1-benzopyran-8-acetic acid as a solid: M.P. 154-159° C.

### Example 7: Preparation of 4-oxo-2-(2,6-difluorophenyl)-4H-1-benzopyran-8-acetic acid.

### Step 1

The same procedure as used in Example 4 was used to give 8-allyl-2-(2,6-difluorophenyl)-4H-benzopyran-4-one as pale yellow crystals in 35% yield: M.P. 84-86° C; TLC R_{f} 0.45 in 10:1 hexane-ethyl acetate.

### Step 2

A similar procedure as used to prepare 4-oxo-2-(2-fluorophenyl)-4H-1-benzopyran-8-acetic acid in Example 4 was used, employing 6 equivalents of sodium periodate and 3.3 mol % of ruthenium trichloride for 1.5 hours at 0° C. to give 4-oxo-2-(2,6-difluorophenyl)-4H-1-benzopyran-8-acetic acid in 35% yield as white crystals: M.P. 178-180° C; TLC R_{f} 0.27 in 40:40:1 hexane-ethyl acetate-acetic acid.

### Example 8: Preparation of 4-oxo-2-(3,5-difluorophenyl)-4H-1-benzopyran-8-acetic acid.

### Step 1

The same procedure as used in Example 4 was used to prepare 8-allyl-2-(3,5-difluorophenyl)-4H-1-benzopyran-4-one in 38% yield as white crystals: M.P. 152-155° C; TLC R_{f} 0.35 in 10:1 hexane-ethyl acetate.

### Step 2

A similar procedure as used to prepare 4-oxo-2-(2-fluorophenyl)-4H-1-benzopyran-8-acetic acid in Example 4 was used, employing 5 equivalents of sodium periodate and 3.3 mol % of ruthenium trichloride for 1.5 hours at room temperature to give 4-oxo-2-(3,5-difluorophenyl)-4H-1-benzopyran-8-acetic acid as white crystals in 16% yield: M.P. 221-223° C; TLC R_{f} 0.29 in 40:40:1 hexane-ethyl acetate-acetic acid.

### Example 9: Preparation of 4-oxo-2-(2,5-difluorophenyl)-4H-1-benzopyran-8-acetic acid

To a 500 ml flask was added 20 g (165.3 mmol) allylbromide, 20 g (147 mmol) 2-hydroxyacetophenone, 10 g (178.6 mmol) potassium hydroxide and 250 ml acetone. The mixture was stirred and refluxed for 6 hours. The resulting suspension was filtered, and the filtered solid was washed with ethyl acetate (3 x 100 ml). The filtrate was evaporated in vacuum to give 27.8 g of an allyl ether as a yellow oil. The crude allyl ether was directly heated neat at 220°C (oven temperature) for 72 hours under N₂. The reaction product was collected as a pale yellow oil after vacuum distillation (b.p. 105-115°C at 1 mm) to give 24.4 g (94%) of 3-allyl-2-hydroxyacetophenone.

To a 100 ml flask fitted with a calcium chloride drying tube was added 5.5 g (31.2 mmol) of 3-allyl-2-hydroxyacetophenone, 5 g (28.4 mmol) of 2,5-difluorobenzoyl chloride, 5 ml of Et₃N and 10 ml of pyridine. The temperature of the reaction mixture rose spontaneously. The stirred reaction mixture was heated at 70°C for one hour. The mixture was cooled and poured into 200 ml of 5% HCl with stirring and extracted with EtOAc (3 x 50 ml). The combined organic layers were washed with H₂O and dried over MgSO₄, and then concentrated to give 9.5 g of 2,5-difluoro-flavone acetic acid as a yellow crude oil. The crude product was directly used in next step without further purification.

To a one liter round-bottom flask containing 200 ml of CH₃CN, 200 ml of CCl₄ and 300 ml of water was added 9.4 g (28.4 mmol) of crude 2,5-difluoro-flavone acetic acid, 31.1 g (5.12 eq) of NalO₄ and 290 mg (0.044 eq) of RuCl₃·H₂O. After stirring vigorously for 2.5 hours at room temperature, the organic layer was separated, and the aqueous layer was extracted with CH₂Cl₂ (100 ml x 3). The combined organic layers were washed with 10% aq Na₂SO₃ solution (50 ml) and brine, dried over MgSO₄, and concentrated to give 10.5 g of crude acid product.

To a warmed 15 ml pyridine solution of 10.5 g (28.4 mmol) of the crude acid product was added 4.97 g (80%, 2.5 eq) of pulverized KOH. The resulting mixture was heated to 70°C and stirred for 1 hour during which time much precipitate formed. The mixture was cooled and acidified with 250 ml of 5% aqueous HCl solution. The crude diketone formed was separated as yellow precipitate which was collected on a filter and dried to give 5.2 g (54%) of crude product.

To a solution of 5.2 g of the crude diketone in 50 ml of glacial acetic acid was added 2 ml of concentrated H₂SO₄ with stirring. The resulting mixture was heated to 100°C for 1 hour and then poured onto 300 g ice with vigorous stirring. The crude final product was collected on a filter, washed with H₂O (300 ml) and recrystallized in MeOH to give 1.58 g of 4-oxo-2-(2,5-difluorophenyl)-4H-1-benzopyran-8-acetic acid as white crystals with an overall yield of 17% from 2,5-difluorobenzoyl-chloride. M.P. 223 ± 1°C

### FORMULAS

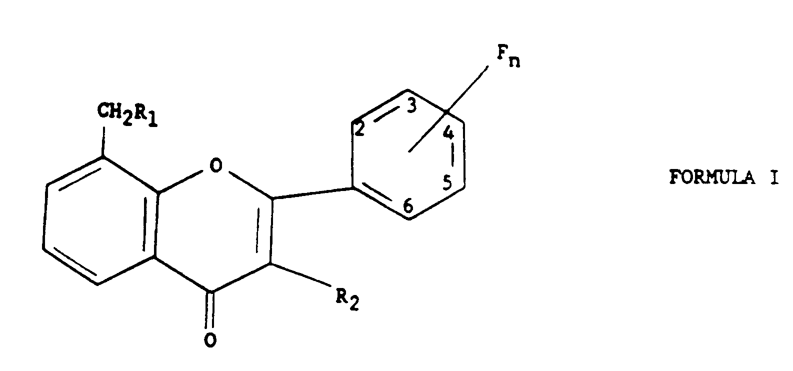

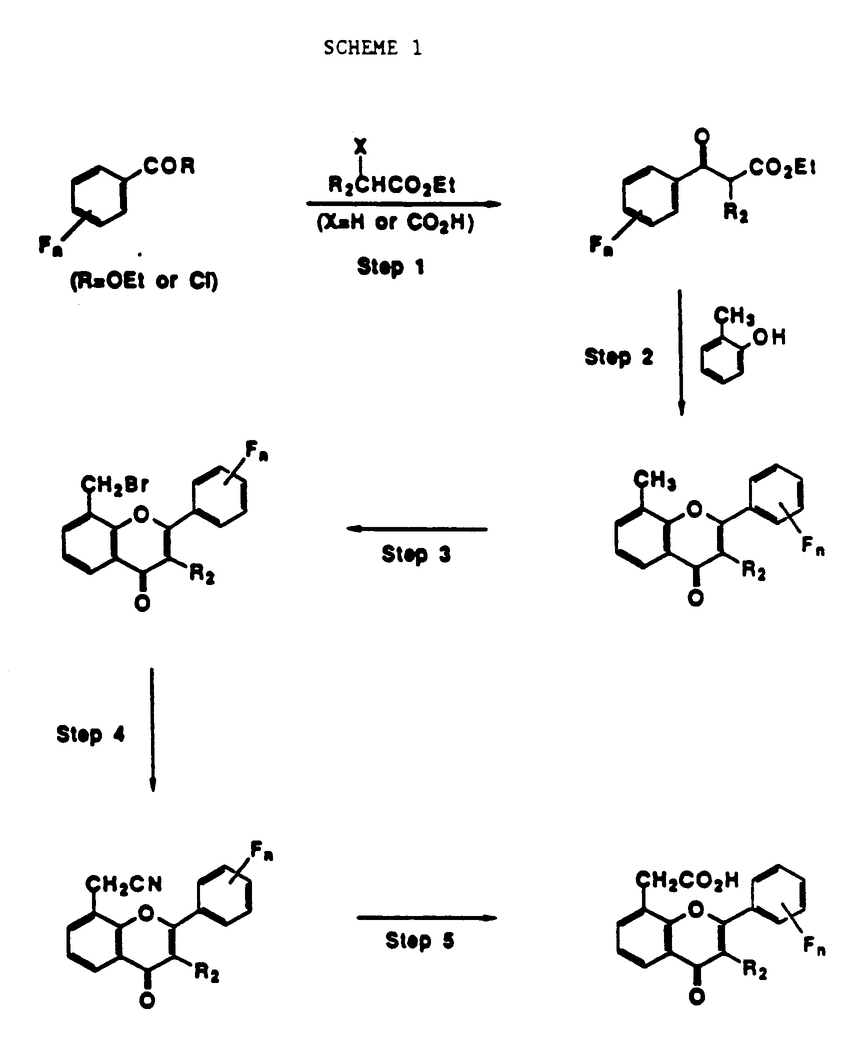

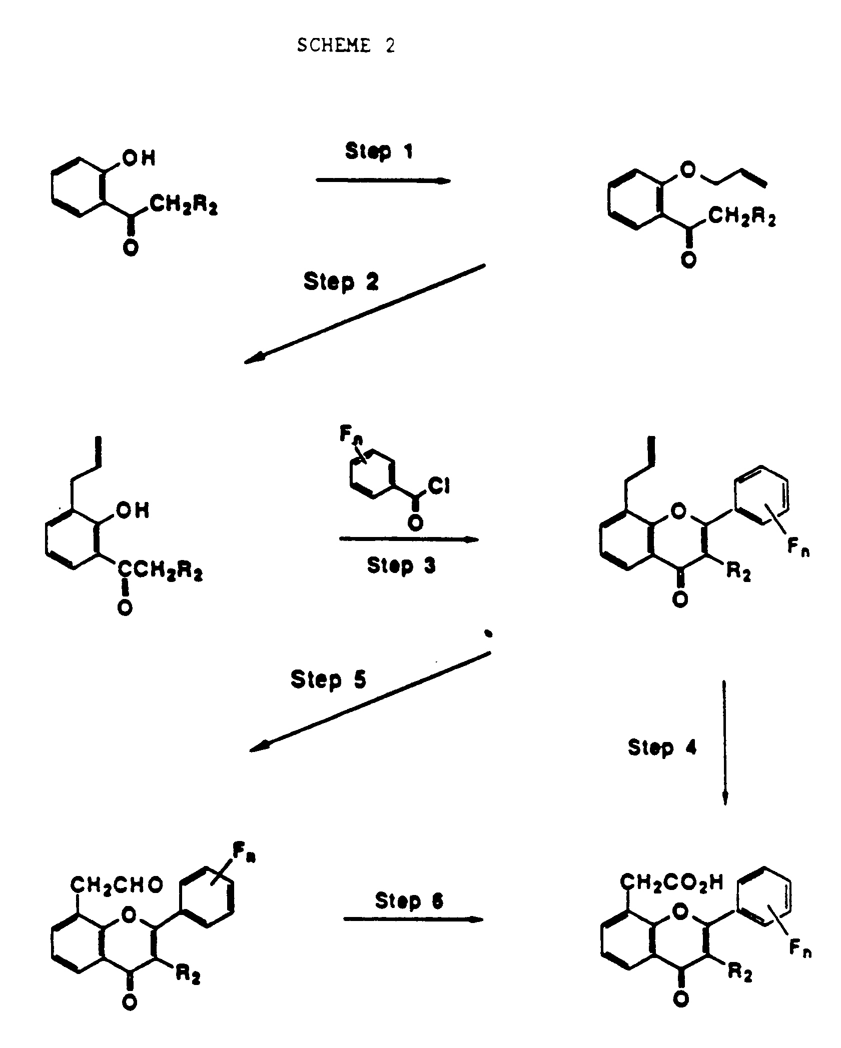

## Claims (Claims for the following Contracting State(s): AT, BE, CH, DE, FR, GB, IT, LI, LU, NL, SE)

1. A compound comprising: 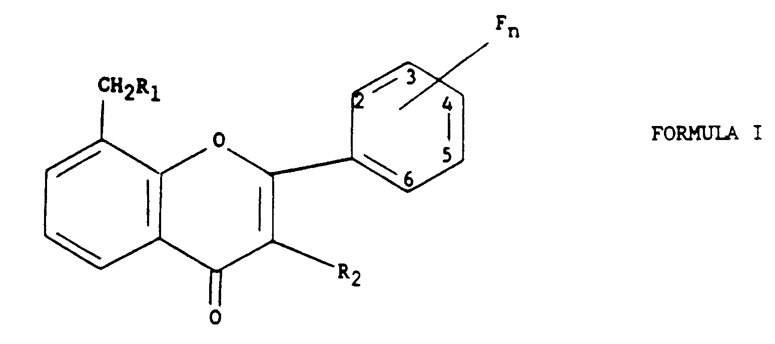 wherein R₁ is CHO, CN, CO₂M, CO₂R₃ or CONR₃R₄ where M is hydrogen or a pharmaceutically acceptable salt;
R₂ is hydrogen, fluorine, methyl, CF₃, phenyl or substituted phenyl;
R₃ and R₄ are independently hydrogen, C₁-C₁₂ alkyl or heterosubstituted alkyl, C₃-C₁₀ cycloalkyl or heterosubstituted cycloalkyl, C₆-C₁₂ aryl, C₇-C₂₄ alkylaryl or are joined to form a C₃-C₁₀ cycloalkyl or heterosubstituted cycloalkyl: and
n is 1 to 5, inclusive.

2. The compound of Claim 1 wherein R₁ is CO₂M.

3. The compound of Claim 2 wherein R₂ is hydrogen.

4. The compound of Claim 1 wherein said fluorine is positioned on said phenyl to form:
a) 2-fluorophenyl;
b) 2,6-difluorophenyl;
c) 2,3,4,5-tetrafluorophenyl;
d) 3-fluorophenyl;
e) 3,4-difluorophenyl;
f) 3,5-difluorophenyl;
g) 4-fluorophenyl;
h) pentafluorophenyl;
i) 3,4,5-trifluorophenyl;
j) 2,3,5,6-tetrafluorophenyl;
k) 2,3-difluorophenyl;
l) 2,3,4-trifluorophenyl;
m) 2,4,6-trifluorophenyl;
n) 2,5-difluorophenyl; or
o) 2,4-difluorophenyl.

5. The compound of Claim 1 which is:
a) 4-oxo-2-(2-fluorophenyl)-4H-1-benzopyran-8-acetic acid;
b) 4-oxo-2-(2,6-difluorophenyl)-4H-1-benzopyran-8-acetic acid;
c) 4-oxo-2-(2,3,4,5-tetrafluorophenyl)-4H-1-benzopyran-8-acetic acid;
d) 4-oxo-2-(3-fluorophenyl)-4H-1-benzopyran-8-acetic acid;
e) 4-oxo-2-(4-fluorophenyl)-4H-1-benzopyran-8-acetic acid;
f) 4-oxo-2-(3,5-difluorophenyl)-4H-1-benzopyran-8-acetic acid;
g) 4-oxo-2-(3,4-difluorophenyl)-4H-1-benzopyran-8-acetic acid;
h) 4-oxo-2-(pentafluorophenyl)-4H-1-benzopyran-8-acetic acid;
i) 4-oxo-2-(3,4,5-trifluorophenyl)-4H-1-benzopyran-8-acetic acid;
j) 4-oxo-2-(2,3,5,6-tetrafluorophenyl)-4H-1-benzopyran-8-acetic acid;
k) 4-oxo-2-(2,3-difluorophenyl)-4H-1-benzopyran-8-acetic acid;
l) 4-oxo-2-(2,3,4-trifluorophenyl)-4H-1-benzopyran-8-acetic acid;
m) 4-oxo-2-(2,4,6-trifluorophenyl)-4H-1-benzoypran-8-acetic acid;
n) 4-oxo-2-(2,5-difluorophenyl)-4H-1-benzopyran-8-acetic acid; or
o) 4-oxo-2-(2,4-difluorophenyl)-4H-1-benzopyran-8-acetic acid.

6. A pharmaceutical composition comprising a compound of any of claims 1 to 5, and a pharmaceutically-acceptable carrier.

7. Use of a compound of any of claims 1 to 5, for the manufacture of a medicament for use in treating tumours.

## Claims (Claims for the following Contracting State(s): ES)

1. A process for preparing a compound of formula I 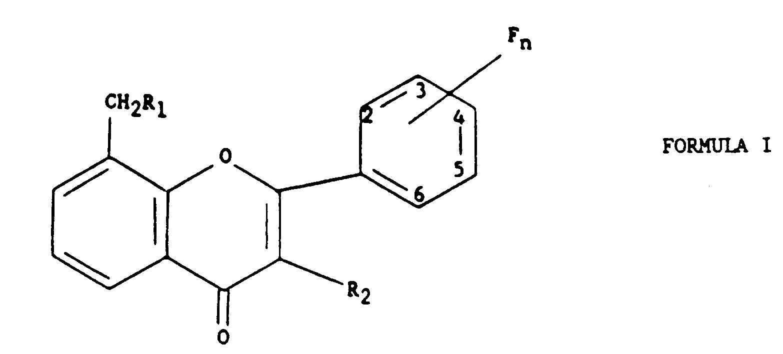 wherein R₁ is CHO or COOH;
R₂ is hydrogen, fluorine, methyl, CF₃, phenyl or substituted phenyl; and
n is 1 to 5;
which comprises oxidising a corresponding compound in which R₁ represents -CH=CH₂.

2. A process according to claim 1, wherein R₁ is CHO and the oxidising is accomplished by ozonolysis or osmium tetroxide/sodium periodate or with RuCl₃/NaIO₄.

3. A process according to claim 1, wherein R₁ is COOH and the oxidising comprises using RuCl₃, NaIO₄ or NaIO₄, KMnO₄ or ozonolysis followed by ozonide oxidation with hydrogen peroxide.

4. A process for preparing a compound of formula I as defined in claim 1 wherein R₁ is COOH, which comprises oxidising the corresponding compound in which R₁ is CHO.

5. A process according to claim 4, wherein the oxidising comprises sodium chlorite oxidation or silver oxide oxidation.

6. A process for preparing a compound of formula I as defined in claim 1 except that R₁ is CN, which comprises converting a corresponding compound in which R₁ represents Br.

7. A process according to claim 6, wherein the conversion comprises a displacement using potassium cyanide and potassium iodide.

8. A process for preparing a compound of formula I as defined in claim 1 wherein R₁ is COOH, which comprises hydrolysing a corresponding compound in which R₁ is CN.

9. A process according to claim 8, wherein the hydrolysing comprises using acetic acid, water and sulphuric acid.

10. A process for preparing a compound of formula I as defined in claim 1 except that R₁ is a pharmaceutically-acceptable salt form of COOH, CO₂R₃ or CONR₃R₄ where R₃ and R₄ are independently hydrogen, C₁-C₁₂ alkyl or heterosubstituted alkyl, C₃-C₁₀ cycloalkyl or heterosubstituted cycloalkyl, C₆-C₁₂ aryl, C₇-C₂₄ alkylaryl or are joined to form a C₃-C₁₀ cycloalkyl or heterosubstituted cycloalkyl, which comprises converting by known methods a corresponding compound in which R₁ is COOH.

11. A process according to any preceding claim, wherein R₂ is hydrogen.

12. A process according to any preceding claim, wherein Fₙ is positioned, on the phenyl group to which it is attached, to form:
a) 2-fluorophenyl;
b) 2,6-difluorophenyl;
c) 2,3,4,5-tetrafluorophenyl;
d) 3-fluorophenyl;
e) 3,4-difluorophenyl;
f) 3,5-difluorophenyl;
g) 4-fluorophenyl;
h) pentafluorophenyl;
i) 3,4,5-trifluorophenyl;
j) 2,3,5,6-tetrafluorophenyl;
k) 2,3-difluorophenyl;
l) 2,3,4-trifluorophenyl;
m) 2,4,6-trifluorophenyl;
n) 2,5-difluorophenyl; or
o) 2,4-difluorophenyl.

13. A process according to claim 12, wherein the compound is:
a) 4-oxo-2-(2-fluorophenyl)-4H-1-benzopyran-8-acetic acid;
b) 4-oxo-2-(2,6-difluorophenyl)-4H-1-benzopyran-8-acetic acid;
c) 4-oxo-2-(2,3,4,5-tetrafluorophenyl)-4H-1-benzopyran-8-acetic acid;
d) 4-oxo-2-(3-fluorophenyl)-4H-1-benzopyran-8-acetic acid;
e) 4-oxo-2-(4-fluorophenyl)-4H-1-benzopyran-8-acetic acid;
f) 4-oxo-2-(3,5-difluorophenyl)-4H-1-benzopyran-8-acetic acid;
g) 4-oxo-2-(3,4-difluorophenyl)-4H-1-benzopyran-8-acetic acid;
h) 4-oxo-2-(pentafluorophenyl)-4H-1-benzopyran-8-acetic acid;
i) 4-oxo-2-(3,4,5-trifluorophenyl)-4H-1-benzopyran-8-acetic acid;
j) 4-oxo-2-(2,3,5,6-tetrafluorophenyl)-4H-1-benzopyran-8-acetic acid;
k) 4-oxo-2-(2,3-difluorophenyl)-4H-1-benzopyran-8-acetic acid;
l) 4-oxo-2-(2,3,4-trifluorophenyl)-4H-1-benzopyran-8-acetic acid;
m) 4-oxo-2-(2,4,6-trifluorophenyl)-4H-1-benzoypran-8-acetic acid;
n) 4-oxo-2-(2,5-difluorophenyl)-4H-1-benzopyran-8-acetic acid; or
o) 4-oxo-2-(2,4-difluorophenyl)-4H-1-benzopyran-8-acetic acid.

## Patentansprüche (Patentansprüche für folgende(n) Vertragsstaat(en): AT, BE, CH, DE, FR, GB, IT, LI, LU, NL, SE)

1. Verbindung entsprechend 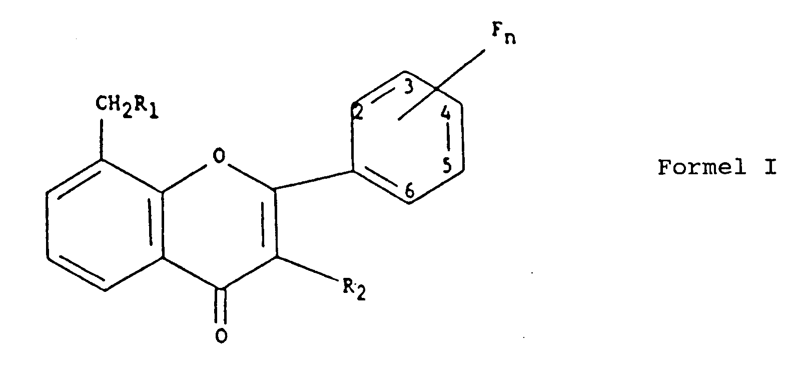 worin bedeuten:
R₁ CHO, CN, CO₂M, CO₂R₃ oder CONR₃R₄ mit M gleich Wasserstoff oder einem pharmazeutisch akzeptablen Salz und R₃ und R₄ unabhängig voneinander gleich Wasserstoff, C₁-C₁₂ Alkyl oder heterosubstituiertem Alkyl, C₃-C₁₀ Cycloalkyl oder heterosubstituiertem Cycloalkyl, C₆-C₁₂ Aryl oder C₇-C₂₄ Alkylaryl oder zusammen gleich C₃-C₁₀ Cycloalkyl oder heterosubstituiertem Cycloalkyl;
R₂ Wasserstoff, Fluor, Methyl, CF₃, Phenyl oder substituiertes Phenyl und
n 1 bis einschließlich 5

2. Verbindung nach Anspruch 1 mit R₁ gleich CO₂M.

3. Verbindung nach Anspruch 2 mit R₂ gleich Wasserstoff.

4. Verbindung nach Anspruch 1, wobei die Fluorsubstitution am Phenyl derart ist, daß
a) 2-Fluorphenyl;
b) 2,6-Difluorphenyl;
c) 2,3,4,5-Tetrafluorphenyl;
d) 3-Fluorphenyl;
e) 3,4-Difluorphenyl;
f) 3,5-Difluorphenyl;
g) 4-Fluorphenyl;
h) Pentafluorphenyl;
i) 3,4,5-Trifluorphenyl;
j) 2,3,5,6-Tetrafluorphenyl;
k) 2,3-Difluorphenyl;
l) 2,3,4-Trifluorphenyl;
m) 2,4,6-Trifluorphenyl;
n) 2,5-Difluorphenyl oder
o) 2,4-Difluorphenyl
gebildet wird.

5. Verbindung nach Anspruch 1, nämlich
a) 4-Oxo-2-(2-fluorphenyl)-4H-1-benzopyran-8-essigsäure
b) 4-Oxo-2-(2,6-difluorphenyl)-4H-1-benzopyran-8-essigsäure
c) 4-Oxo-2-(2,3,4,5-tetrafluorphenyl)-4H-1-benzopyran-8-essigsäure
d) 4-Oxo-2-(3-fluorphenyl)-4H-1-benzopyran-8-essigsäure
e) 4-Oxo-2-(4-fluorphenyl)-4H-1-benzopyran-8-essigsäure
f) 4-Oxo-2-(3,5-difluorphenyl)-4H-1-benzopyran-8-essigsäure
g) 4-Oxo-2-(3,4-difluorphenyl)-4H-1-benzopyran-8-essigsäure
h) 4-Oxo-2-(pentafluorphenyl)-4H-1-benzopyran-8-essigsäure
i) 4-Oxo-2-(3,4,5-trifluorphenyl)-4H-1-benzopyran-8-essigsäure
j) 4-Oxo-2-(2,3,5,6-tetrafluorphenyl)-4H-1-benzopyran-8-essigsäure
k) 4-Oxo-2-(2,3-difluorphenyl)-4H-1-benzopyran-8-essigsäure
l) 4-Oxo-2-(2,3,4-trifluorphenyl)-4H-1-benzopyran-8-essigsäure
m) 4-Oxo-2-(2,4,6-trifluorphenyl)-4H-1-benzopyran-8-essigsäure
n) 4-Oxo-2-(2,5-difluorphenyl)-4H-1-benzopyran-8-essigsäure oder
o) 4-Oxo-2-(2,4-difluorphenyl)-4H-1-benzopyran-8-essigsäure.

6. Arzneimittelzubereitung mit einer Verbindung nach einem der Ansprüche 1 bis 5 und einem pharmazeutisch akzeptablen Träger.

7. Verwendung einer Verbindung nach einem der Ansprüche 1 bis 5 zur Herstellung eines Medikaments zur Verwendung bei der Tumorbehandlung.

## Patentansprüche (Patentansprüche für folgende(n) Vertragsstaat(en): ES)

1. Verfahren zur Herstellung einer Verbindung der Formel I 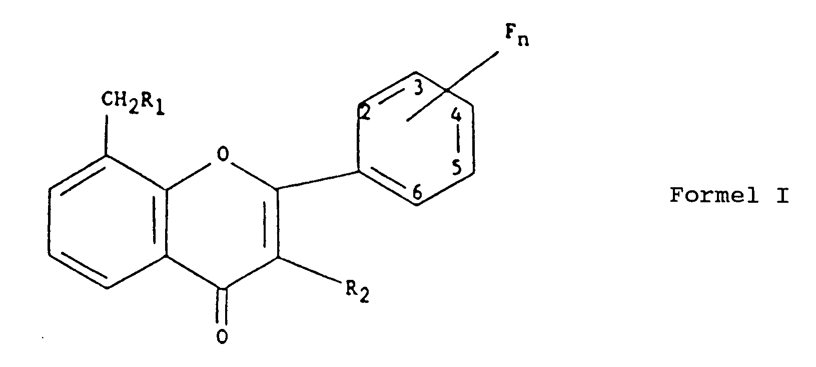 worin bedeuten:
R₁ CHO oder COOH;
R₂ Wasserstoff, Fluor, Methyl, CF₃, Phenyl oder substituiertes Phenyl und
n 1 bis 5
durch Oxidieren einer entsprechenden Verbindung, worin R₁ für -CH=CH₂ steht.

2. Verfahren nach Anspruch 1, wobei R₁ für CHO steht und die Oxidation durch Ozonolyse oder Osmiumtetraoxid/Natriumperjodat oder mit RuCl₃/NaIO₄ bewerkstelligt wird.

3. Verfahren nach Anspruch 1, wobei R₁ für COOH steht und die Oxydation unter Verwendung von RuCl₃, NaIO₄ oder NaIO₄, KMnO₄ oder Ozonolyse und anschließende Ozonidoxidation mit Wasserstoffperoxid bewerkstelligt wird.

4. Verfahren zur Herstellung einer Verbindung der Formel I gemäß Anspruch 1, worin R₁ für COOH steht, durch Oxidieren der entsprechenden Verbindung mit R₁ gleich CHO.

5. Verfahren nach Anspruch 4, wobei die Oxidation eine Natriumchloritoxidation oder Silberoxidoxidation umfaßt.

6. Verfahren zur Herstellung einer Verbindung der Formel I gemäß Anspruch 1, mit der Ausnahme, daß R₁ = CN, durch Umwandlung einer entsprechenden Verbindung mit R₁ gleich Br.

7. Verfahren nach Anspruch 6, wobei die Umwandlung in einer Verdrängung unter Verwendung von Kaliumcyanid und Kaliumjodid besteht.

8. Verfahren zur Herstellung einer Verbindung der Formel I gemäß Anspruch 1, mit R₁ gleich COOH, durch Hydrolyse einer entsprechenden Verbindung mit R₁ gleich CN.

9. Verfahren nach Anspruch 8, wobei die Hydrolyse unter Verwendung von Essigsäure, Wasser und Schwefelsäure erfolgt.

10. Verfahren zur Herstellung einer Verbindung der Formel I gemäß Anspruch 1, mit der Ausnahme, daß R₁ für eine pharmazeutisch akzeptable Salzform von COOH, CO₂R₃ oder CONR₃R₄ mit R₃ und R₄ unabhängig jeweils gleich Wasserstoff, C₁-C₁₂ Alkyl oder heterosubstituiertem Alkyl, C₃-C₁₀ Cycloalkyl oder heterosubstituiertem Cycloalkyl, C₆-C₁₂ Aryl oder C₇-C₂₄ Alkylaryl oder gemeinsam gleich C₃-C₁₀ Cycloalkyl oder heterosubstituiertem Cycloalkyl steht, durch Umwandeln einer entsprechenden Verbindung mit R₁ gleich COOH.

11. Verfahren nach einem der vorhergehenden Ansprüche, wobei R₂ für Wasserstoff steht.

12. Verfahren nach einem der vorhergehenden Ansprüche, wobei Fₙ an der Phenylgruppe, an der es hängt, derart positioniert ist, daß
a) 2-Fluorphenyl;
b) 2,6-Difluorphenyl;
c) 2,3,4,5-Tetrafluorphenyl;
d) 3-Fluorphenyl;
e) 3,4-Difluorphenyl;
f) 3,5-Difluorphenyl;
g) 4-Fluorphenyl;
h) Pentafluorphenyl;
i) 3,4,5-Trifluorphenyl;
j) 2,3,5,6-Tetrafluorphenyl;
k) 2,3-Difluorphenyl;
l) 2,3,4-Trifluorphenyl;
m) 2,4,6-Trifluorphenyl;
n) 2,5-Difluorphenyl oder
o) 2,4-Difluorphenyl
gebildet wird.

13. Verfahren nach Anspruch 12, wobei die Verbindung aus
a) 4-Oxo-2-(2-fluorphenyl)-4H-1-benzopyran-8-essigsäure
b) 4-Oxo-2-(2,6-difluorphenyl)-4H-1-benzopyran-8-essigsäure
c) 4-Oxo-2-(2,3,4,5-tetrafluorphenyl)-4H-1-benzopyran-8-essigsäure
d) 4-Oxo-2-(3-fluorphenyl)-4H-1-benzopyran-8-essigsäure
e) 4-Oxo-2-(4-fluorphenyl)-4H-1-benzopyran-8-essigsäure
f) 4-Oxo-2-(3,5-difluorphenyl)-4H-1-benzopyran-8-essigsäure
g) 4-Oxo-2-(3,4-difluorphenyl)-4H-1-benzopyran-8-essigsäure
h) 4-Oxo-2-(pentafluorphenyl)-4H-1-benzopyran-8-essigsäure
i) 4-Oxo-2-(3,4,5-trifluorphenyl)-4H-1-benzopyran-8-essigsäure
j) 4-Oxo-2-(2,3,5,6-tetrafluorphenyl)-4H-1-benzopyran-8-essigsäure
k) 4-Oxo-2-(2,3-difluorphenyl)-4H-1-benzopyran-8-essigsäure
l) 4-Oxo-2-(2,3,4-trifluorphenyl)-4H-1-benzopyran-8-essigsäure
m) 4-Oxo-2-(2,4,6-trifluorphenyl)-4H-1-benzopyran-8-essigsäure
n) 4-Oxo-2-(2,5-difluorphenyl)-4H-1-benzopyran-8-essigsäure oder
o) 4-Oxo-2-(2,4-difluorphenyl)-4H-1-benzopyran-8-essigsäure
besteht.

## Revendications (Revendications pour l'(les) Etat(s) contractant(s) suivant(s): AT, BE, CH, DE, FR, GB, IT, LI, LU, NL, SE)

1. Composé de formule 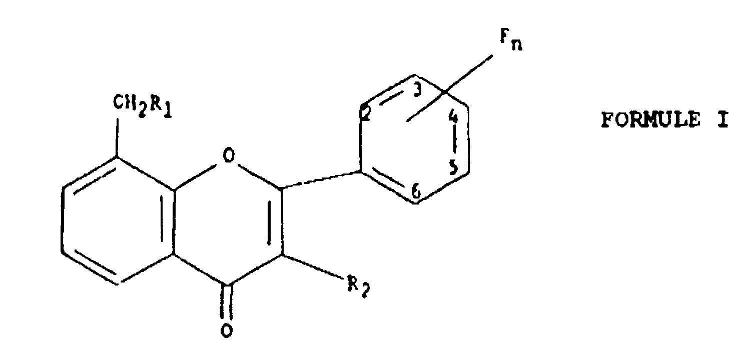 dans laquelle R₁ représente un groupe CHO, CN, CO₂M, CO₂R₃ ou CONR₃R₄ dans lequel M représente l'hydrogène, ou un sel pharmaceutiquement acceptable ;
R₂ représente l'hydrogène, le fluor, un groupe méthyle, CF₃, phényle ou phényle substitué ;
R₃ et R₄ représentent indépendamment l'hydrogène, un groupe alkyle ou alkyle hétérosubstitué en C₁ à C₁₂, cycloalkyle ou cycloalkyle hétérosubstitué en C₃ à C₁₀, aryle en C₆ à C₁₂, alkylaryle en C₇ à C₂₄, ou bien sont joints pour former un groupe cycloalkyle en cycloalkyle hétérosubstitué en C₃ à C₁₀ ; et
n a une valeur de 1 à 5 inclus.

2. Composé suivant la revendication 1, dans lequel R₁ représente un groupe CO₂M.

3. Composé suivant la revendication 2, dans lequel R₂ représente l'hydrogène.

4. Composé suivant la revendication 1, dans lequel le fluor est positioné sur le groupe phényle pour former :
a) un groupe 2-fluorophényle ;
b) un groupe 2,6-difluorophényle ;
c) un groupe 2,3,4,5-tétrafluorophényle ;
d) un groupe 3-fluorophényle ;
e) un groupe 3,4-difluorophényle ;
f) un groupe 3,5-difluorophényle ;
g) un groupe 4-fluorophényle ;
h) un groupe pentafluorophényle ;
i) un groupe 3,4,5-trifluorophényle ;
j) un groupe 2,3,5,6-tétrafluorophényle ;
k) un groupe 2,3-difluorophényle ;
l) un groupe 2,3,4-trifluorophényle ;
m) un groupe 2,4,6-trifluorophényle ;
n) un groupe 2,5-difluorophényle ; ou
o) un groupe 2,4-difluorophényle.

5. Composé suivant la revendication 1, qui est :
a) l'acide 4-oxo-2-(2-fluorophényl)-4H-1-benzopyranne-8-acétique ;
b) l'acide 4-oxo-2-(2,6-difluorophényl)-4H-1-benzopyranne-8-acétique ;
c) l'acide 4-oxo-2-(2,3,4,5-tétrafluorophényl)-4H-1-benzopyranne-8-acétique ;
d) l'acide 4-oxo-2-(3-fluorophényl)-4H-1-benzopyranne-8-acétique ;
e) l'acide 4-oxo-2-(4-fluorophényl)-4H-1-benzopyranne-8-acétique ;
f) l'acide 4-oxo-2-(3,5-difluorophényl)-4H-1-benzopyranne-8-acétique ;
g) l'acide 4-oxo-2-(3,4-difluorophényl)-4H-1-benzopyranne-8-acétique ;
h) l'acide 4-oxo-2-(pentafluorophényl)-4H-1-benzopyranne-8-acétique ;
i) l'acide 4-oxo-2-(3,4,5-trifluorophényl)-4H-1-benzopyranne-8-acétique ;
j) l'acide 4-oxo-2-(2,3,5,6-tétrafluorophényl)-4H-1-benzopyranne-8-acétique ;
k) l'acide 4-oxo-2-(2,3-difluorophényl)-4H-1-benzopyranne-8-acétique ;
l) l'acide 4-oxo-2-(2,3,4-triflurophényl)-4H-1-benzopyranne-8-acétique ;
m) l'acide 4-oxo-2-(2,4,6-trifluorophényl)-4H-1-benzopyranne-8-acétique ;
n) l'acide 4-oxo-2-(2,5-diflurophényl)-4H-1-benzopyranne-8-acétique ; ou
o) l'acide 4-oxo-2-(2,4-difluorophényl)-4H-1-benzopyranne-8-acétique.

6. Composition pharmaceutique comprenant un composé suivant l'une quelconque des revendications 1 à 5, et un support pharmaceutiquement acceptable.

7. Utilisation d'un composé suivant l'une quelconque des revendications 1 à 5, pour la production d'un médicament destiné à être utilisé dans le traitement de tumeurs.

## Revendications (Revendications pour l'(les) Etat(s) contractant(s) suivant(s): ES)

1. Procédé de préparation d'un composé de formule 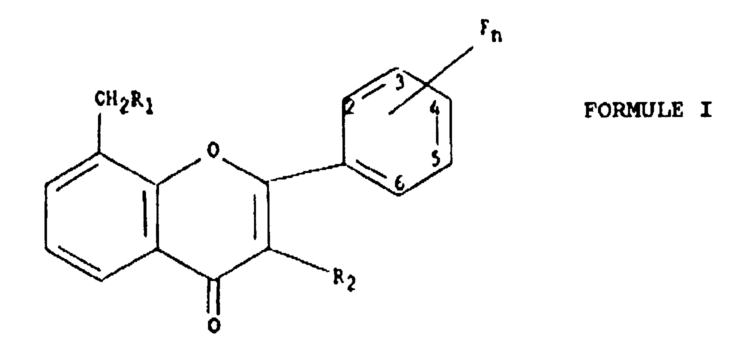 dans laquelle R₁ représente un groupe CHO ou COOH ;
R₂ représente l'hydrogène, le fluor, un groupe méthyle CF₃, phényle ou phényle substitué ; et
n a une valeur de 1 à 5 ;
qui comprend l'oxydation d'un composé correspondant dans lequel R₁ représente un groupe -CH=CH₂.

2. Procédé suivant la revendication 1, dans lequel R₁ représente un groupe CHO et l'oxydation est effectuée par ozonolyse ou action du tétroxyde de sodium/periodate de sodium ou de RuCl₃/NaIO₄.

3. Procédé suivant la revendication 1, dans lequel R₁ représente un groupe COOH et l'oxydation comprend l'utilisation de RuCl₃, NaIO₄ ou NaIO₄, KMnO₄, ou bien une ozonolyse suivie par l'oxydation de l'ozonide avec le peroxyde d'hydrogène.

4. Procédé de préparation d'un composé de formule I suivant la revendication 1, dans lequel R₁ représente un groupe COOH, qui comprend l'oxydation du composé correspondant, dans lequel R₁ représente un groupe CHO.

5. Procédé suivant la revendication 4, dans lequel l'oxydation comprend une oxydation avec le chlorite de sodium ou une oxydation avec l'oxyde d'argent.

6. Procédé de préparation d'un composé de formule I suivant la revendication 1, sauf que R₁ représente un groupe CN, qui comprend la transformation d'un composé correspondant dans lequel R₁ représente Br.

7. Procédé suivant la revendication 6, dans lequel la transformation comprend un déplacement au moyen de cyanure de potassium et d'iodure de potassium.

8. Procédé de préparation d'un composé de formule I suivant la revendication 1, dans lequel R₁ représente un groupe COOH, qui comprend l'hydrolyse d'un composé correspondant dans lequel R₁ représente un groupe CN.

9. Procédé suivant la revendication 8, dans lequel l'hydrolyse comprend l'utilisation d'acide acétique, d'eau et d'acide sulfurique.

10. Procédé de préparation d'un composé de formule I suivant la revendication 1, sauf que R₁ représente un sel pharmaceutiquement acceptable du groupe COOH, un groupe CO₂R₃ ou CONR₃R₄ dans lequel R₃ et R₄ représentent indépendamment l'hydrogène, un groupe alkyle ou alkyle hétérosubstitué en C₁ à C₁₂, cycloalkyle ou cycloalkyle hétérosubstitué en C₃ à C₁₀, aryle en C₆ à C₁₂, alkylaryle en C₇ à C₂₄ ou bien sont joints pour former un groupe cycloalkyle ou cycloalkyle hétérosubstitué en C₃ à C₁₀, qui comprend la transformation d'un composé correspondant dans lequel R₁ représente un groupe COOH.

11. Procédé suivant l'une quelconque des revendications précédentes, dans lequel R₂ représente l'hydrogène.

12. Procédé suivant l'une quelconque des revendications précédentes, dans lequel Fₙ est positionné, sur le groupe phényle auquel il est fixé, pour former
a) un groupe 2-fluorophényle ;
b) un groupe 2,6-difluorophényle ;
c) un groupe 2,3,4,5-tétrafluorophényle ;
d) un groupe 3-fluorophényle ;
e) un groupe 3,4-difluorophényle ;
f) un groupe 3,5-difluorophényle ;
g) un groupe 4-fluorophényle ;
h) un groupe pentafluorophényle ;
i) un groupe 3,4,5-trifluorophényle ;
j) un groupe 2,3,5,6-tétrafluorophényle ;
k) un groupe 2,3-difluorophényle ;
l) un groupe 2,3,4-trifluorophényle ;
m) un groupe 2,4,6-trifluorophényle ;
n) un groupe 2,5-difluorophényle; ou
o) un groupe 2,4-difluorophényle.

13. Procédé suivant la revendication 12, dans lequel le composé est ;
a) l'acide 4-oxo-2-(2-fluorophényl)-4H-1-benzopyranne-8-acétique ;
b) l'acide 4-oxo-2-(2,6-difluorophényl)-4H-1-benzopyranne-8-acétique ;
c) l'acide 4-oxo-2-(2,3,4,5 tétrafluorophényl)-4H-1-benzopyranne-8-acétique ;
d) l'acide 4-oxo-2-(3-fluorophényl)-4H-1-benzopyranne-8-acétique ;
e) l'acide 4-oxo-2-(4-fluorophényl)-4H-1-benzopyranne-8-acétique ;
f) l'acide 4-oxo-2-(3,5-difluorophényl)-4H-1-benzopyranne-8-acétique ;
g) l'acide 4-oxo-2-(3,4-difluorophényl)-4H-1-benzopyranne-8-acétique ;
h) l'acide 4-oxo-2-(pentafluorophényl)-4H-1-benzopyranne-8-acétique ;
i) l'acide 4-oxo-2-(3,4,5-trifluorophényl)-4H-1-benzopyranne-8-acétique ;
j) l'acide 4-oxo-2-(2,3,5,6-tétrafluorophényl)-4H-1-benzopyranne-8-acétique ;
k) l'acide 4-oxo-2-(2,3-difluorophényl)-4H-1-benzopyranne-8-acétique ;
l) l'acide 4-oxo-2-(2,3,4-trifluorophényl)-4H-1-benzopyranne-8-acétique ;
m) l'acide 4-oxo-2-(2,4,6-trifluorophényl)-4H-1-benzopyranne-8-acétique ;
n) l'acide 4-oxo-(2,5-difluorophényl)-4H-1-benzopyranne-8-acétique ; ou
o) l'acide 4-oxo-2-(2,4-difluorophényl)-4H-1-benzopyranne-8-acétique.
